## Europäisches Patentamt

## European Patent Office

## Office européen des brevets

(11) Veröffentlichungsnummer: 0 365 831

A1

(12) EUROPÄISCHE PATENTANMELDUNG

(21) Anmeldenummer: 89117536.6

(51) Int. Cl.5: B01F 1/00 , B01F 3/08 , B01F 13/06

(22) Anmeldetag: 22.09.89

(30) Priorität: 30.09.88 DE 3833230

(43) Veröffentlichungstag der Anmeldung:
02.05.90 Patentblatt 90/18

(84) Benannte Vertragsstaaten:
ES GR

(71) Anmelder: Henkel Kommanditgesellschaft auf Aktien
Henkelstrasse 67
D-4000 Düsseldorf 13(DE)

(72) Erfinder: Körner, Hermann
Kamper Weg 292
D-4000 Düsseldorf 12(DE)
Erfinder: Grimm, Klaus-Dieter
von Felbrückstrasse 31
D-4018 Langenfeld(DE)
Erfinder: Hill, Karlheinz, Dr.
Am Hasenbusch 1
D-4006 Erkrath(DE)

(54) Verfahren zum Lösen hochschmelzender Erzeugnisse in tiefsiedenden Flüssigkeiten als Lösungsmittel, insbesondere mit simultaner Bleiche.

(57) Zur Herstellung von insbesondere homogenen Pasten wird der zu lösende Feststoff über seinen Festpunkt erwärmt und die als Lösungsmittel dienende Flüssigkeit unter solchen Bedingungen hinzugefügt, daß in der Vermischungsvorrichtung ein Betriebsdruck von 1,1 bis 10 bar entsteht und eine Verdampfung des Lösungsmittels verhindert wird. Das Verfahren kann diskontinuierlich oder kontinuierlich durchgeführt werden. Der Löse- bzw. Anpastungsprozeß kann mit einem Bleichprozeß verbunden werden. Als Lösungsmittel wird bevorzugt Wasser verwendet; der Bleichprozeß wird vorzugsweise mit Wasserstoffperoxid durchgeführt. Das Verfahren läßt sich generell auf solche Erzeugnisse der organischen Chemie anwenden, die einen Stockpunkt im Bereich von etwa 50 °C bis 250 °C aufweisen, und die zum Zwecke einer besseren Handhabung in gelöste bzw. pastenartige Form überführt werden können.

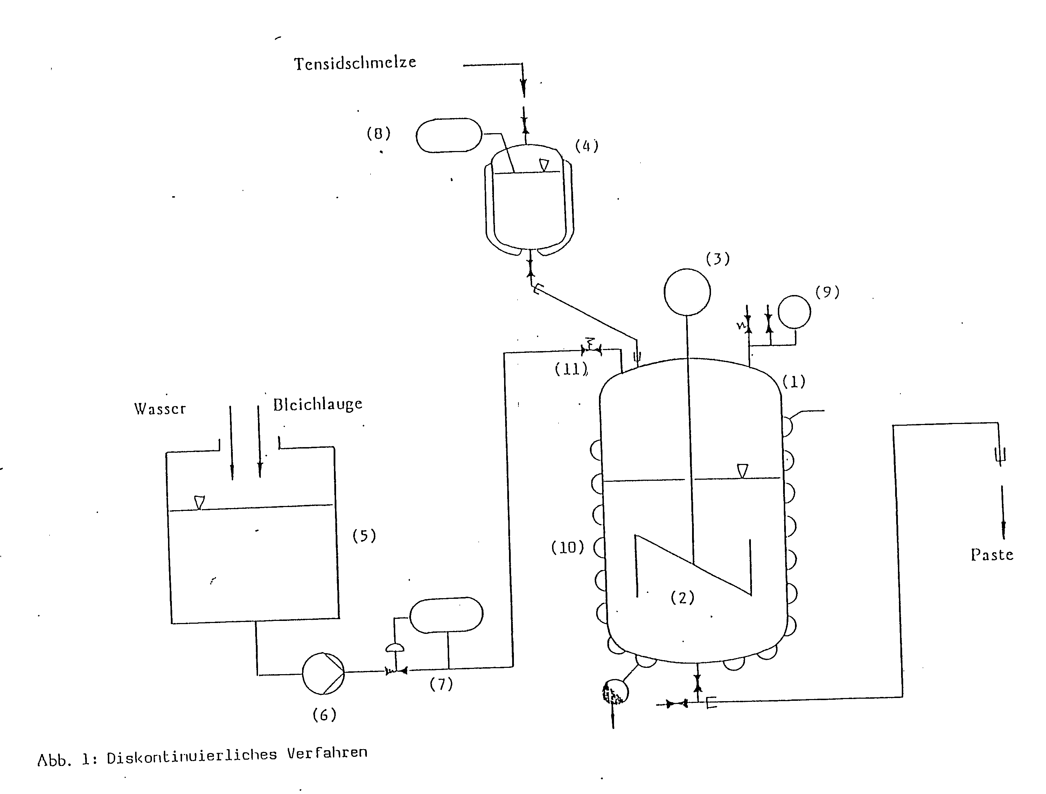

Abb. 1: Druckontinuierliches Verfahren

## Verfahren zum Lösen hochschmelzender Erzeugnisse in tiefsiedenden Flüssigkeiten als Lösungsmittel, insbesondere mit simultaner Bleiche

Die Erfindung betrifft ein Verfahren zum Lösen von hochschmelzenden Erzeugnissen in tiefsiedenden Flüssigkeiten als Lösungsmittel. Unter dem Begriff Lösen wird das homogene Verteilen des hochschmelzenden Erzeugnisses mit und in dem tiefsiedenden Lösungsmittel verstanden, wobei in Abhängigkeit von der Konzentration des Endprodukts dieser Begriff von molekulardisperser Lösung bis homogener, leicht verdünnbarer Paste reicht. Unter hochschmelzenden Erzeugnissen werden solche Stoffe der organischen Chemie verstanden, deren Schmelzpunkte bzw. Festpunkte bzw. Stockpunkte über 50 °C, insbesondere über 100 °C und ganz besonders über 120 °C liegen. Festpunktsobergrenzen spielen bei dem Verfahren keine Rolle; allerdings ergibt sich aus der Natur der eingesetzten organisch-chemischen Verbindungen, die im wesentlichen monomolekulare Strukturen haben, ein Maximalwert für den Festpunkt von ca. 250 °C. Als tiefsiedende Flüssigkeiten werden solche Lösungsmittel bezeichnet, die zum Lösen des festen Stoffes geeignet sind und die beim Festpunkt der hochschmelzenden Verbindung bereits einen Dampfdruck von über 100 mbar aufweisen. Insbesondere weisen diese Lösungsmittel einen Siedepunkt auf, der bei Normaldruck unterhalb des Festpunkts des hochschmelzenden Erzeugnisses, und der insbesondere um mindestens 10 °C darunter liegt. In der Europäischen Patentanmeldung 0 165 721 A1 wird ein Verfahren zum Bleichen von organischen Erzeugnissen, insbesondere von Alkylpolyglykosiden beschrieben, bei dem man das Erzeugnis zunächst mit Wasserstoffperoxid und anschließend mit einer Schwefeldioxid freisetzenden Substanz behandelt, um auf diese Weise zu farbstabilen Produkten zu gelangen. Dieses Behandlungsverfahren wird an wäßrigen Pasten des organischen Erzeugnisses ausgeübt, ohne daß die Herstellung dieser Pasten näher beschrieben wird.

In der US-amerikanischen Patentschrift 3,839,318 wird die Herstellung von Alkylglucosiden im Labormaßstab (mit 2 Mol Glucose) beschrieben. Dabei wird das Reaktionsprodukt direkt nach Abdestillieren des Fettalkohol-Überschusses in der Schmelze bei 135 °C über einen auf den Reaktionskolben aufgesetzten Kühler mit Wasser versetzt und so eine Paste mit 70 % Feststoff und einem pH-Wert von 5,8 erhalten (Spalte 6, Zeilen 56 bis 62). Nach den Angaben an anderer Stelle dieser Veröffentlichung wird eine derartige wäßrige Paste mit pH-Wert 4,3 mit Natriumperborat gebleicht und so ein gebleichtes Produkt mit pH 6,1 erhalten. Es liegt auf der Hand, daß diese Verfahrensschritte des Überführens in eine Paste (Anpasten) und des Bleichens sich nicht auf großtechnische Ansätze im 100-kg- bis Tonnenmaßstab übertragen lassen.

Wenn bei einem Herstellungsverfahren im großtechnischen Maßstab, eine Produktschmelze in einem niedrigsiedenden Lösungsmittel, gemäß obiger Definition, gelöst werden soll, dann können dabei erhebliche Probleme auftreten. Diese Probleme reichen vom teilweisen Verdampfen des Lösungsmittels und den daraus resultierenden Nachteilen wie Belastung der Umwelt, Rezepturfehler, erhöhte Kosten, bis zum ungewollten Erstarren der Produktschmelze beim Vermischen mit dem Lösungsmittel, was zu verlängerten Rührzeiten, zu ungewollten Inhomogenitäten und damit zu erschwerter Handhabbarkeit führen kann. Zusätzlich können, wenn es sich bei dem Produkt um einen Tensidrohstoff handelt, beim Rühren und bei einer Bleichbehandlung erhebliche Schaumprobleme auftreten.

Diese und andere Nachteile können vermieden bzw. überwunden werden, wenn man bei der Handhabung großtechnischer Produktmengen dafür sorgt, daß man den Lösungsvorgang unter Überdruck durchführt.

Demnach ist das Verfahren zum Lösen hochschmelzender Erzeugnisse in tiefsiedenden Lösungsmitteln, insbesondere mit simultaner Bleiche, dadurch gekennzeichnet, daß man das zu lösende Erzeugnis bei einer Temperatur über seinem Festpunkt (Stockpunkt) hält und die als Lösungsmittel dienende Flüssigkeit so hinzufügt, daß in der Vermischungsvorrichtung ein Betriebsdruck von 1,1 bis 10 bar bei der Zugabe des Lösungsmittels entsteht und eine Verdampfung des Lösungsmittels beim Vermischen danach nicht mehr auftritt.

Mit dem erfindungsgemäßen Verfahren können zwar echte molekulardisperse Lösungen hergestellt werden, bevorzugt ist jedoch die Herstellung von homogenen Pasten, die sich zur Weiterverarbeitung zu sowohl flüssigen als auch festen Konfektionsformen eignen.

Vorzugsweise stellt man homogene Pasten mit einem Feststoffgehalt (Produktmenge) von 20 - 80 Gew.-%, vorzugsweise 40 - 70 Gew.-% her. Das erfindungsgemäße Verfahren kann diskontinuierlich oder kontinuierlich durchgeführt werden. Die Vorteile des erfindungsgemäßen Verfahrens sind mehrfacher Art: Während des Vermischungsvorganges wird die Bildung einer festen Phase vermieden; das Verfahren führt zu einer erheblichen Verkürzung der Lösezeit; Verluste an Lösungsmitteln durch Verdampfen und damit verbundene Rezepturfehler, Umweltbelastungen und Kosten werden vermieden.

Ein weiterer besonderer Vorteil des Verfahrens liegt darin, daß der Löseprozeß mit einem Bleichprozeß verbunden werden kann. Handelt es sich beim Lösen und Bleichen um an sich schäumende Tensidrohstoffe, so können die Schaumprobleme während des Verfahrens gut beherrscht werden. Damit stellt das erfindungsgemäße Verfahren eine Problemlösung für die ökonomische Handhabung und Verarbeitung im betrieblichen Produktionsmaßstab dar.

Die Erfindung betrifft insbesondere das Überführen von wachsartigen bis festen Erzeugnissen, die sich für die Weiterverarbeitung zu Waschmitteln oder kosmetischen Mitteln eignen, insbesondere fettchemische Produkte, in hochkonzentrierte Pasten mit geringer Eigenfärbung und guter Lagerstabilität. Dabei ist die Herstellung von alkoholischen, wäßrig/alkoholischen und wäßrigen Pasten besonders bevorzugt. Das Verfahren bezieht sich in besonderer Weise auf das Überführen der durch Säurekatalyse aus Glykosen, Oligoglykosen oder Polyglykosen hergestellten Alkylglykoside, insbesondere Fettalkylglucoside, in wäßrige, insbesondere 40 - 70 %ige Pasten bei gleichzeitiger Bleiche mit oxidierenden Substanzen, insbesondere Peroxyverbindungen, so daß hellfarbige Pasten resultieren, die sich auf Grund ihrer Farbstabilität im alkalischen Milieu und ihrer Lagerstabilität zur Herstellung von Wasch- und Reinigungsmitteln eignen.

Nach einer bevorzugten Ausführungsform des Verfahrens wird als Lösungsmittel Wasser verwendet und das Löseverfahren mit einem Bleichprozeß unter Verwendung einer Aktivsauerstoff abgebenden Verbindung, insbesondere Wasserstoffperoxid, durchgeführt. Dabei werden bevorzugt Betriebsdrucke von 2 bis 10 bar eingestellt. Prinzipiell kann dieser Bleichprozeß auch unter Verwendung einer aktivchlorhaltigen bzw. -freisetzenden Verbindung durchgeführt werden; jedoch ist diese Variante wegen der unvermeidlichen Bildung von Chloridionen, die in vielen Fällen bei der Anwendung des gebleichten Produkts zu Korrosionsproblemen führen können, weniger bevorzugt. Das erfindungsgemäße Verfahren läßt sich generell auf solche Erzeugnisse der organischen Chemie anwenden, die einen Stockpunkt im Bereich von etwa 50 °C bis 250 °C, vorzugsweise 50 bis 200 °C, aufweisen, und die zum Zwecke einer besseren Handhabung in gelöste bzw. pastenartige Form überführt werden können. Aus anwendungstechnischer Sicht werden von derartigen Lösungen oder Pasten häufig bestimmte Eigenschaften zu ihrer Farb- bzw. Lagerstabilität verlangt, so daß eine Bleichung des Erzeugnisses notwendig wird.

Das erfindungsgemäße Verfahren läßt sich prinzipiell auf solche Produkte der Chemischen Industrie anwenden, die bei ihrer Herstellung als Schmelzen anfallen und beim Abkühlen in feste bzw. wachsartige bzw. gelartige Konsistenz übergehen, insbesondere auf fettchemische Produkte. Solche Produkte werden häufig zur besseren Handhabung und Weiterverarbeitung in Lösungen oder Pasten übergeführt. Typische Vertreter für diese Substanzen sind die Partialester von langkettigen Fettsäuren mit Glycerin, die Ester von langkettigen Fettsäuren mit Ethylenglykolen, die Fettalkohole, die Fettalkoholpolyglykolether, die Kondensationsprodukte von langkettigen Fettsäuren mit Polyaminoalkanen bzw. Hydroxyalkylpolyaminoalkanen oder quartäre Ammoniumverbindungen mit einem oder zwei langkettigen Alkylresten.

Als besonders bevorzugte Ausführungsform wird das erfindungsgemäße Verfahren als kombiniertes Löse- und Bleichverfahren unter Verwendung von Wasser als Lösungsmittel und Wasserstoffperoxid als Oxidationsmittel zum Aufarbeiten von großtechnischen Mengen an Alkylglucosiden, vornehmlich den Fettalkylglucosiden, die als nichtionische Tenside für die Herstellung von flüssigen und pulverförmigen Waschmitteln und kosmetischen Mitteln geeignet sind, angewendet.

Alkylglykoside und ihre Herstellung werden außer in den obengenannten Dokumenten beispielsweise in der US-Patentschrift 3 547 828 bzw. in den europäischen Patentanmeldungen 0 035 589 A1 und 092 355 A1 beschrieben. Bei der Alkylkomponente der Alkylglykoside handelt es sich generell um aliphatische Reste mit $C_1$ bis $C_{30}$, von denen der Bereich $C_8$ bis $C_{20}$, insbesondere $C_{12}$ bis $C_{18}$, Produkte mit Tensideigenschaften ergibt. Die Zuckerkomponente im Alkylglykosid kann prinzipiell von üblichen Aldosen bzw. Ketosen abstammen. Wegen der in großen Mengen verfügbaren Glucose und ihrer guten Reaktionsfähigkeit sind die Alkylglucoside besonders bevorzugt. Typische Vertreter der Alkylglykoside, welche die Eigenschaften eines nichtionischen Tensids besitzen, sind solche, deren aliphatischer Rest einen typischen Fettalkylrest, beispielsweise also Octyl, Decyl, Dodecyl, Tetradecyl, Hexadecyl, Octadecyl darstellt. Besonders geeignete Alkylglykoside enthalten einen Kokosfettalkylrest, d. h. Mischungen mit im wesentlichen Dodecyl und Tetradecyl. Gut geeignet sind als nichtionische Tenside auch solche Alkylglykoside, deren Alkylrest sich von synthetischen primären Alkoholen, insbesondere den sogenannten $C_9$-$C_{13}$-Oxoalkoholen, d. h. solchen primären Alkanolen, die einen gewissen Prozentsatz an verzweigten Isomeren aufweisen, ableitet.

Bei der diskontinuierlichen Arbeitsweise wird die Produktschmelze in einem vorgeheizten Druckkessel vorgelegt, dabei werden Temperaturen, die circa 15 - 25 °C über dem Stockpunkt des festen Produkts liegen, gewählt. In das geschmolzene Produkt wird dann das Lösungsmittel, beispielsweise Wasser, unter langsamen Rühren zudosiert.

Durch das anfänglich verdampfende Lösungsmittel baut sich der erforderliche Betriebsdruck von selbst auf, wobei in Abhängigkeit von der Wahl des Lösungsmittels Drucke von 1,1 bar bis 10 bar auftreten. Im Falle von Wasser als Lösungsmittel entstehen im allgemeinen Drucke von 3,5 bis 5 bar . Nach Beendigung des Vermischungsvorganges und nach der Kühlung der Lösung auf etwa 50 °C sinkt der Betriebsdruck wieder nahezu auf Umgebungsdruck ab. Die Lösungsmittelmenge wird so gewählt, daß eine Lösungsmittelkonzentration zwischen 30 und 90, vorzugsweise zwischen 30 und 70 und insbesondere zwischen 40 und 60 Gew.-% erreicht wird. Bei der Verwendung von Wasser als Lösungsmittel wurde überraschenderweise gefunden, daß das Wasser nicht vorgeheizt werden muß. Es wurde beobachtet, daß der Stockpunkt bereits nach Zumischung von geringen Wassermengen stark absinkt.

Wenn eine Bleichung des Produkts vorgesehen ist, kann die bleichwirksame Substanz, vorzugsweise Wasserstoffperoxid, dem Wasser direkt zugesetzt werden, so daß die Vorgänge des Lösens und des Bleichens gleichzeitig, d. h. ohne zusätzlichen Zeitaufwand für das Bleichen stattfinden. Für den Bleichprozeß werden im allgemeinen solche Mengen der bleichwirksamen Aktivsauerstoffverbindungen, bezogen auf das zu bleichende Produkt verwendet, daß sie 0,2 bis 1,5 Gew.-% berechnet als reines $H_2O_2$, angewendet in üblicher Konzentration (ca. 30 bis 35%ig), entsprechen. Zusätzlich wird so viel Natronlauge zugesetzt, daß die Bleiche deutlich im alkalischen Bereich, vorzugsweise bei pH 7,5 bis pH 10 und insbesondere bei 8 bis 9,5 stattfindet. Hierbei ist darauf zu achten und möglichst in einem Vorversuch zu klären, ob während des Bleichens der pH-Wert absinkt, so daß ein Zusatz an Natriumhydroxid auch dann angezeigt ist, wenn das Produkt vor dem Bleichen bereits alkalisch reagiert hat. Ebenfalls möglich ist eine separate Zugabe der Bleichsubstanz und der Base zu der bereits gekühlten Paste bzw. Lösung des Produkts. Die besten Bleichergebnisse werden bei einer Temperatur von etwa 90 °C und einer Bleichzeit von 0,5 - 4 Stunden erhalten. Um diese optimalen Verfahrensbedingungen einzustellen ist es zweckmäßig, unmittelbar nach Beginn der Zugabe des Wassers bzw. Wasser/Bleichmittels, mit der Kühlung zu beginnen. Für das nach dem erfindungsgemäßen Verfahren in Lösung bzw. in Pastenform übergeführte gebleichte Produkt wird auf diese Weise als Maß für die Farbaufhellung eine Farbzahl nach Klett von unterhalb 35 gefunden. Eine schematische Darstellung für den diskontinuierlichen Verfahrensablauf findet sich in Abb. 1.

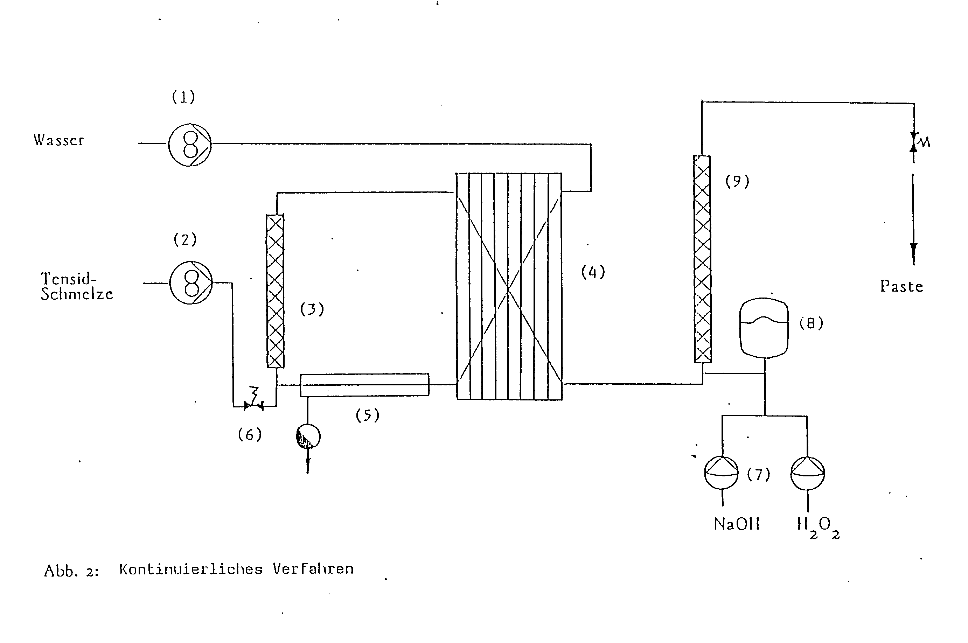

Bei der kontinuierlichen Durchführung des Verfahrens werden das geschmolzene Produkt und das Lösungsmittel, vorzugsweise Wasser, im Mengenverhältnis von 40 % bis 70 %, vorzugsweise 50 % bis 60 % Lösungsmittel, vorzugsweise mit Zahnradpumpen auf den Löserdruck von vorzugsweise etwa 3,5 bis 5 bar gefördert und in einem statischen Mischer homogenisiert. Die heiße Lösung wird in einem Wärmeaustauscher im Gegenstrom zum Lösungsmittel auf 70 - 100 °C, vorzugsweise etwa 90 °C, gekühlt und nach Beendigung des Löseverfahrens bzw. des gleichzeitigen Bleichens auf Umgebungsdruck gebracht. Das im Wärmeaustauscher aufgeheizte Lösungsmittel wird vor dem Eintritt in die Mischstrecke in einem Nacherhitzer auf die gewünschte Lösetemperatur eingestellt, beispielsweise auf etwa 140 °C für das bevorzugt verwendete Lösungsmittel Wasser. Im Falle von Wasser als Lösungsmittel kann auch mit einer wäßrigen Lösung der Aktivsauerstoffverbindung, insbesondere Wasserstoffperoxid, und Natriumhydroxid gleichzeitig gebleicht werden, wobei man die Perverbindung und das Alkali dem Lösewasser vor Eintritt in die Mischstrecke zudosiert. Vorzugsweise wird jedoch eine zweite Mischvorrichtung verwendet, um damit die Bleichchemikalien in die bereits gekühlte Lösung bzw. Paste bei optimaler Bleichtemperatur einzubringen. Das geschieht mit üblichen Dosierpumpen, beispielsweise Membrandosierpumpen, wobei vorzugsweise mit einem Pulsationsdämpfer für einen gleichmäßigen Additivfluß gesorgt wird. Nach einer besonders bevorzugten Verfahrensweise wird der Zusatz der Bleichchemikalien so gesteuert, daß die eigentliche Bleiche im wesentlichen Umfang erst nach dem Lösungsvorgang stattfindet, wenn die auf optimale Bleichtemperatur gehaltene Lösung bzw. Paste transportiert wird bzw. lagert, so daß gegenüber dem diskontinuierlichen Verfahren mit einem reduzierten Volumen der Mischvorrichtungen gearbeitet werden kann. Zusätzlich wurde gefunden, daß bei der kontinuierlichen Verfahrensweise die thermische Belastung für die Produktschmelze geringer ist, so daß man bereits vor der Bleiche hellere Pasten erhält. Eine schematische Darstellung des kontinuierlichen Verfahrens findet sich in Abb. 2.

Die Wirksamkeit des kombinierten Löse/Bleich-Verfahrens wird durch den folgenden Lager- und Alkalistabilitätstest für die wäßrige Paste festgestellt:
Eine Probemenge der wäßrigen Paste wird mit konzentrierter Natronlauge auf den pH-Wert 12 - 13 eingestellt und 0,5 Stunden lang auf 100 °C erhitzt. Nach dem Abkühlen auf Raumtemperatur wird durch Zugabe von Wasser/Isopropylalkohol, Verhältnis ca. 1 : 1, eine 5%ige Lösung eingestellt und die Farbzahl nach Klett gemessen. Dazu wird eine 1-cm-Küvette und ein Blaufilter verwendet. Die Klettzahlen für das so behandelte Produkt sollen unterhalb von 35 liegen.

Beispiel 1

Dieses Beispiel beschreibt das diskontinuierliche Verfahren für einen technischen Ansatz. Eine Menge von 88 kg eines $C_{12}/C_{14}$-Fettalkylglucosids, das nach der Butanol-Umacetalisierungsmethode aus wasserfreier Glucose hergestellt worden war, Stockpunkt des Produkts 135 °C, wurde im geschmolzenen Zustand in einen auf 150 °C vorgeheizten 250 Liter-Druckkessel vorgelegt. Der pH-Wert des Produkts, gemessen an einer 1 %igen wäßrigen Probe, lag bei 9. Wasser von Raumtemperatur in einer Menge von 86 kg (Liter) wurde in einem Zeitraum von 15 Minuten unter langsamen Umrühren (circa 75 U/min) hinzugefügt, wobei sich eine Mischungstemperatur von zunächst 140 °C einstellte, die durch Kühlen auf 90 - 93 °C gebracht wurde. Bei der Wasserzugabe hatte sich ein Betriebsdruck von 3,6 bar aufgebaut. Dem Lösewasser waren vorab 2 kg 30 %iges Wasserstoffperoxid und 2 kg 50 %ige NaOH zugesetzt worden. Der Ansatz wurde 3 Stunden lang bei circa 90 °C langsam gerührt und dann auf 50 °C abgekühlt, danach war der Druck im Reaktor auf Umgebungsdruck gesunken. Die so erhaltene Paste mit ca. 50 Gew.-% Wasser hatte ein hellgelbes trübes Aussehen. Die Viskosität bei Raumtemperatur betrug 30 mPas. Mit dieser Paste wurde der Farbstabilitätstest wie oben angegeben durchgeführt. Die Farbzahlen nach Klett wurden mit 20 (nach der Bleiche) bzw. 25 (nach dem Farbstabilitätstest) gefunden. pH-Wert der Paste 9,5.

Beispiel 2

Dieses Beispiel beschreibt das kontinuierliche Verfahren in einer Pilot-Anlage. Die Produktschmelze des $C_{12}$-$C_{14}$-Alkylglykosids wie in Beispiel 1 wurde im Mengenverhältnis 1 : 1 mit Wasser von Raumtemperatur über die Zahnradpumpen (1) bzw. (2) auf einen Löserdruck von 4,5 bar gefördert und im statischen Mischer (3), Bautype Sulzer SMX, homogenisiert. Die Durchlaufmengen betrugen jeweils 32 kg pro Stunde. Im Plattenwärmeaustauscher (4) wurde die Mischung auf 90 °C abgekühlt und anschließend auf Umgebungsdruck gebracht. Das im Plattenwärmeaustauscher aufgeheizte Lösewasser war vor dem Eintritt in die Mischstrecke auf 140 °C mittels Nacherhitzer (5) gebracht worden. Anschließend durchlief die 90 °C warme Mischung den 2. statischen Mischer des selben Typs und dort hinein wurden über die Membrandosierpumpen (7) und den Pulsationsdämpfer (8) jeweils 0,75 kg 30%iges Wasserstoffperoxid bzw. 50%iges Natriumhydroxid pro Stunde zugeführt. Die fertige Mischung wurde ohne weitere Kühlung über eine Zwischenvorlage im Transportgebinde abgelassen, so daß die Bleichreaktion dort ihren Abschluß finden konnte. Wassergehalt der Paste ca. 52 Gew.-%, Aussehen hellgelbtrüb; Viskosität bei 25 °C 280 mPas. Farbstabilitätstest: Farbzahlen nach Klett 21 nach der Bleiche bzw. 24 nach dem Farbstabilitätstest. pH-Wert der Paste 9,6.

Erläuterungen zu Abb. 1:

(1) Rührbehälter
(2) Rührer
(3) Getriebemotor
(4) Vorlage für Tensidschmelze
(5) Vorlage Anpastwasser/Bleichlauge
(6) Kreiselpumpe
(7) Ringkolbenzähler (selbstabschaltend bei vorgegebener Wassermenge)
(8) Füllstandsalarm
(9) Sicherheits- und Anzeigearmaturen
(10) Halbrohrschlagen zur Dampfbeheizung/Wasserkühlung
(11) Rückschlagventil

Erläuterungen zu Abb. 2:

(1) Zahnradpumpe
(2) Zahnradpumpe, beheizt mit Dampf
(3) Statischer Mischer zur Anpastung
(4) Plattenwärmetauscher zur Aufheizung des Anpastwassers gegen die Paste
(5) Nacherhitzer für Anpastwasser, beheizt mit Dampf
(6) Druckhalteventil
(7) Membrandosierpumpen für Bleich-Chemikalien
(8) Pulsationsdämpfer
(9) Statischer Mischer zur Einarbeitung der Additive in die Paste

**Ansprüche**

1. Verfahren zum Lösen von hochschmelzenden Erzeugnissen in tiefsiedenden Flüssigkeiten als Lösungsmitteln, dadurch gekennzeichnet, daß man das zu lösende Erzeugnis bei einer Temperatur über seinem Festpunkt (Stockpunkt) hält und die als Lösungsmittel dienende Flüssigkeit so hinzufügt, daß in der Vermischungsvorrichtung ein Betriebsdruck von 1,1 bis 10 bar bei der Zugabe des Lösungsmittels entsteht und eine Verdampfung des Lösungsmittels beim Vermischen danach nicht mehr auftritt.

2. Verfahren nach Anspruch 1, dadurch gekennzeichnet, daß man die Menge des Lösungsmittels so wählt, daß man homogene Pasten mit einem Feststoffgehalt (Produktmenge) von 20 - 80, vorzugsweise 40 - 70 Gew.-% erhält.

3. Verfahren nach einem der Ansprüche 1 und 2, dadurch gekennzeichnet, daß man als Lösungsmittel Wasser verwendet.

4. Verfahren nach einem der Ansprüche 1 bis 3, dadurch gekennzeichnet, daß man als organischen Feststoff ein fettchemisches Produkt verwendet.

5. Verfahren nach einem der Ansprüche 1 bis 4, dadurch gekennzeichnet, daß man als fettchemisches Produkt ein nichtionisches Tensid vom Typ der Alkylglykoside, insbesondere Fettalkylglucoside, verwendet.

6. Verfahren nach einem der Ansprüche 1 bis 5, dadurch gekennzeichnet, daß man das Löseverfahren mit einem gleichzeitigen Bleichprozeß unter Verwendung von Perverbindungen, vorzugsweise Wasserstoffperoxid, verbindet.

7. Verfahren nach einem der Ansprüche 1 bis 6, dadurch gekennzeichnet, daß man das Löse- und Bleichverfahren bei einem deutlich alkalischen pH-Wert, vorzugsweise bei pH 7,5 bis 10 und insbesondere bei pH 8 bis 9,5 durchführt und gegebenenfalls eine pH-Wert-Senkung während des Bleichprozesses durch Zugabe von Alkalimetallhydroxid, vorzugsweise Natriumhydroxid vermeidet.

8. Homogene wäßrige Paste eines hochschmelzenden organischen Erzeugnisses erhältlich nach einem der Ansprüche 1 bis 7, dadurch gekennzeichnet, daß die Paste nach dem Bleichen eine Farbzahl nach Klett von weniger als 35, gemessen nach 0,5-stündigem Erhitzen der Paste auf 100 °C bei pH 12 - 13, gemäß Lager- und Alkalistabilitätstest aufweist.

D 8277 EP

Abb. 1: Diskontinuierliches Verfahren

Wasser

(1)

Tensid-
Schmelze

(2)

(3)

(6)

(5)

(4)

(9)

(8)

Paste

(7)

NaOH    H$_2$O$_2$

Abb. 2:    Kontinuierliches Verfahren

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-C- 404 907 (B. WAESER) <br> * Seite 1, Zeilen 1-15,26-31; Seite 2, Zeilen 23-34; Ansprüche 1,2 * <br> --- | 1,3 | B 01 F 1/00 <br> B 01 F 3/08 <br> B 01 F 13/06 |
| Y | CH-A- 289 063 (R. DECKER et al.) <br> * Seite 1, Zeilen 33-44,52-55; Ansprüche 2,4; Beispiel 1 * | 1,3 | |
| A | | 2 | |
| | --- | | |
| A | DE-A-2 305 411 (HENKEL & CIE GmbH) <br> * Seite 2, Absätze 1,2; Seite 5, letzter Absatz; Seite 6, Absätze 1,2; Ansprüche * <br> --- | 1-4 | |
| A | DE-C- 694 142 (H. PLAUSON) <br> * Seite 1, Zeilen 21-44; Seite 2, Zeilen 8-28; Anspruch * <br> ----- | 6 | |

|  |
|---|
| **RECHERCHIERTE SACHGEBIETE (Int. Cl.5)** |
| B 01 F <br> C 11 D <br> B 01 B |

Der vorliegende Recherchenbericht wurde für alle Patentansprüche erstellt

| Recherchenort | Abschlußdatum der Recherche | Prüfer |
|---|---|---|
| DEN HAAG | 03-01-1990 | MARZENKE J. |

EPO FORM 1503 03.82 (P0403)